(19) Europäisches Patentamt
European Patent Office
Office européen des brevets

(11) **EP 4 447 088 A1**

(12) **EUROPEAN PATENT APPLICATION**

(43) Date of publication:
**16.10.2024 Bulletin 2024/42**

(51) International Patent Classification (IPC):
***H01J 37/32*** *(2006.01)*    ***H05H 1/46*** *(2006.01)*
***A61B 18/04*** *(2006.01)*

(21) Application number: **24167544.6**

(22) Date of filing: **28.03.2024**

(52) Cooperative Patent Classification (CPC):
**H01J 37/32174; A61B 18/042; H05H 1/46;
H05H 1/4645**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **03.04.2023 US 202318130163**

(71) Applicant: **CAPS MEDICAL LTD.
4250547 Netaniya (IL)**

(72) Inventors:
• **YANOVITZ, Leonid**
  **7528442 Rishon LeZion (IL)**
• **UCHITEL, Ilan Oleg**
  **4465139 Kfar-Saba (IL)**
• **KOGAN, Boris**
  **2603797 Kiriat-Motzkin (IL)**

(74) Representative: **Schneiders & Behrendt Bochum
Gerard-Mortier-Platz 6
44793 Bochum (DE)**

(54) **PLASMA GENERATING SYSTEM**

(57)    Power circuitry for cold plasma generation; optionally plasma for therapeutic use. Cold plasma generation occurs at the distal end of a catheter-like device which is flexible, narrow (e.g., less than 5 mm in diameter), and longitudinally extended to reach, e.g., 50-100 cm into body cavities. A cable used for power transmission is a part of the power generating circuit, its intrinsic impedance being a major contributor to and constraint on the time constant of an entraining RC circuit whose resonant frequency entrains the frequency of power generation. In some embodiments, inductive transformer coupling to the entraining/transmission line circuit is used to generate voltage gain. In some embodiments, transformer coupling is divided into a plurality of stages. This potentially enables practically achieving high transmission frequencies with higher gain, lowered sensitivity to variability in distal portions of the entraining RC circuit, and/or longer transmission lines compared to a single-stage transformer configuration.

FIG. 1A

**Description**

FIELD AND BACKGROUND OF THE INVENTION

The present invention, in some embodiments thereof, relates to the field of plasma generation, and more particularly, but not exclusively, to generation and therapeutic delivery of cold plasma.

Non-Thermal Atmospheric Plasma (NTAP), also referred to as Cold Atmospheric Plasma (CAP), is a near room-temperature ionized gas composed of various neutral and charged species. It is widely used in various application fields and industries, including in medicine where its beneficial characteristics are well established. Application of NTAP on tissue exerts complex and unique impact both on cell/tissue level and systemic level.

NTAP may be generated by Dielectric Barrier Discharge (DBD), wherein a flowing gas (typically, a noble gas such as Helium or Argon) is ionized by an electrode upon which high voltage is periodically applied. the electrode is covered by a dielectric barrier, such that no direct ohmic path is generated between the driving source and the applied target tissue.

SUMMARY OF THE INVENTION

According to an aspect of some embodiments of the present disclosure, there is provided power supply circuitry for a non-thermal plasma generator, the circuitry including: a gain transformer including a primary coil and a secondary coil; a driver circuit electrically connected to drive a current through the primary coil; a load circuit having a distal end including a plasma generating site, and a proximal end coupled to the secondary coil of the gain transformer; wherein the load circuit includes at least one decoupling transformer inductively interconnecting the plasma generating site and the secondary coil.

According to some embodiments of the present disclosure, the load circuit has an impedance determining a frequency of its oscillation in response to current generated in the secondary coil.

According to some embodiments of the present disclosure, the load circuit entrains oscillation of the driver circuit.

According to some embodiments of the present disclosure, the load circuit oscillation is entrained via feedback from the gain transformer.

According to some embodiments of the present disclosure, the frequency of oscillation of the load circuit is sufficiently high that plasma generation at the plasma generating site does not extinguish during at least a full oscillation cycle.

According to some embodiments of the present disclosure, the gain transformer provides a gain of at least 20.

According to some embodiments of the present disclosure, the at least one decoupling transformer provides in aggregate a gain no larger than 1.

According to some embodiments of the present disclosure, the at least one decoupling transformer provides in aggregate a gain smaller than the gain provided by the gain transformer by a factor of at least 2.

According to some embodiments of the present disclosure, the gain transformer and at least one coupling transformer comprise air or ferrite cores.

According to some embodiments of the present disclosure, the at least one decoupling transformer includes a plurality of decoupling transformers.

According to some embodiments of the present disclosure, the gain transformer and at least one coupling transformer isolate the plasma generating site from ground.

According to some embodiments of the present disclosure, the load circuit includes: a transmission line, and a distal transformer of the at least one decoupling transformers including a secondary coil of the distal transformer inductively interconnected with a primary coil of the distal transformer and connected to a proximal side of the transmission line; the driver circuit: is electrically interconnected with the proximal side of the transmission line via the primary and secondary coils of the distal transformer, and provides to the transmission line an electrical signal with an operating frequency of between 1-10 MHz, which transmits to a distal end of the transmission line with an operating amplitude of at least 0.5 kV RMS; and wherein impedance on the secondary coil of the distal transformer is small enough in combination with the capacitance of the transmission line that a circuit portion including the transmission line and secondary coil operates in resonance upon receiving the electrical signal.

According to some embodiments of the present disclosure, the driver circuit ceases production of the electrical signal when the transmission line is disconnected, but maintains production of the electrical signal for values of transmission line capacitance varying within a range having at least a 10% difference between its minimum and maximum values.

According to some embodiments of the present disclosure, the operating amplitude is at least 1 kV RMS.

According to some embodiments of the present disclosure, the transmission line is at least 50 cm long, and flexible.

According to some embodiments of the present disclosure, the load circuit provides a feedback signal to the driver circuit via a feedback network.

According to some embodiments of the present disclosure, oscillation of the load circuit entrains the driver circuit, via the feedback network, to produce the electrical signal at the operating frequency.

According to some embodiments of the present disclosure, provided together with a gas supply lumen leading along the transmission line to the plasma generating site, the gas supply lumen and transmission line together being elements of a flexible probe has an overall diameter of less than 10 mm.

According to some embodiments of the present disclosure, the plasma generating site generates non-thermal plasma when powered by the electrical signal.

According to some embodiments of the present disclosure, the at least one decoupling transformer together with the gain transformer comprise a plurality of transformers interconnecting the proximal end of the transmission line to a low voltage signal oscillating at the operating frequency, with a voltage amplitude at least 20 times smaller than the operating amplitude.

According to some embodiments of the present disclosure, the primary coil of the gain transformer has an inductance in the range of about 1-5 μH, and the secondary winding of the gain transformer an inductance in the range of about 1000-5000 μH.

According to some embodiments of the present disclosure, the gain transformer includes a feedback winding providing a feedback signal to the driver circuit via a feedback network, and wherein the feedback winding has an inductance in the range of about 1-10 μH.

According to some embodiments of the present disclosure, the feedback signal entrains oscillation of the driver circuit to the operating frequency, and is received at the feedback winding via the load circuit at a frequency of electrical oscillation of the transmission line.

According to some embodiments of the present disclosure, the secondary winding of the distal transformer has an inductance in the range of about 20-80 μH, and the primary winding of the distal transformer has an inductance in the range of about 5-20 μH.

According to some embodiments of the present disclosure, the secondary winding of the distal transformer is connected to conductors of the transmission line through electrical contacts.

According to some embodiments of the present disclosure, the power supply circuitry includes pulse modulation circuitry operable to modulate the operating frequency at a lower frequency including a frequency in the range of 0.1 - 1 KHz.

According to an aspect of some embodiments of the present disclosure, there is provided a method of decoupling gain and frequency constraints on non-thermal plasma generation, including: providing a site of plasma generation an output electrical signal having an operating frequency and a voltage amplitude sufficient to generate plasma; wherein the site of plasma generation is electrically interconnected via at least two transformer stages to an input electrical signal oscillating at the operating frequency and at least 20 time lower in voltage than the output electrical signal.

According to some embodiments of the present disclosure, the voltage amplitude is at least 1 kV

According to some embodiments of the present disclosure, the operating frequency is between 1-10 MHz.

According to some embodiments of the present disclosure, the site of plasma generation is interconnected with the input electrical signal via a transmission line, and oscillation of a circuit including the transmission line entrains oscillation of the input electrical signal.

According to an aspect of some embodiments of the invention, there is provided a non-thermal plasma generator including: a gain transformer including a primary coil and a secondary coil; a driver circuit electrically connected to drive a current through the primary coil; a load circuit having a distal end including a plasma generating site generating non-thermal plasma, and a proximal end coupled to the secondary coil of the gain transformer; wherein the load circuit includes at least one decoupling inductor, providing inductance including inductance connected in parallel to the secondary coil, and inductance connected in parallel to the plasma generating site.

According to some embodiments of the invention, the load circuit has an impedance determining a frequency of oscillation of the load circuit in response to a current generated in the secondary coil.

According to some embodiments of the invention, the load circuit entrains oscillation of the driver circuit.

According to some embodiments of the invention, oscillation of the driver circuit is entrained via feedback from the gain transformer.

According to some embodiments of the invention, the frequency of oscillation of the load circuit is sufficiently high that plasma generation at the plasma generating site does not extinguish during at least a full oscillation cycle.

According to some embodiments of the invention, the non-thermal plasma generator includes pulse modulation circuitry operable to modulate the frequency of oscillation at a lower frequency within the range of 0.1-1 KHz.

According to some embodiments of the invention, the gain transformer provides a gain of at least 20.

According to some embodiments of the invention, the at least one decoupling inductor includes at least one decoupling transformer, providing in aggregate a gain no larger than 1.

According to some embodiments of the invention, the at least one decoupling inductor includes decoupling transformers providing in aggregate a gain smaller than the gain provided by the gain transformer by a factor of at least 2.

According to some embodiments of the invention, the gain transformer includes air or ferrite core.

According to some embodiments of the invention, the at least one decoupling inductor includes a plurality of decoupling transformers.

According to some embodiments of the invention, the at least one decoupling inductor includes an inductor coil connected both in parallel to the secondary coil, and in parallel to the plasma generating site.

According to some embodiments of the invention, the driver circuit ceases oscillation when the plasma generating site is disconnected from the load circuit, but maintains oscillation for values of the frequency of oscillation of the load circuit varying within a range having at least a 10% difference between minimum and maximum values of the range.

According to some embodiments of the invention, an operating voltage amplitude at the plasma generating site produced when the current is driven through the primary coil of the gain transformer is at least 1 kV RMS.

According to some embodiments of the invention, the transmission line is at least 50 cm long, and flexible.

According to some embodiments of the invention, provided together with a gas supply lumen leading along the transmission line to the plasma generating site, the gas supply lumen and transmission line together being elements of a flexible probe has an overall diameter of less than 10 mm.

According to some embodiments of the invention, the at least one decoupling inductor together with the gain transformer comprise a one or more transformers delivering the operating voltage to the plasma generating site with an amplitude at least 20 times larger than a voltage amplitude in the primary coil of the gain transformer.

According to some embodiments of the invention, the primary coil of the gain transformer has an inductance in the range of about 1-5 $\mu$H, and the secondary coil of the gain transformer has an inductance in the range of about 1000-5000 $\mu$H.

According to some embodiments of the invention, the feedback from the gain transformer is provided by a feedback winding of the gain transformer, and wherein the feedback winding has an inductance in the range of about 1-10 $\mu$H.

According to some embodiments of the invention, the at least one decoupling inductor includes at least one decoupling transformer, and a distal-side coil of the at least one decoupling transformer has an inductance in the range of about 20-80 $\mu$H, and is a coil of a distal decoupling transformer of the at least one decoupling transformer having a primary coil with an inductance in the range of about 5-20 $\mu$H.

According to some embodiments of the invention, the at least one decoupling inductor is connected to the plasma generating site through releasable electrical contacts.

Unless otherwise defined, all technical and/or scientific terms used herein have the same meaning as commonly understood by one of ordinary skill in the art to which the present disclosure pertains. Although methods and materials similar or equivalent to those described herein can be used in the practice or testing of embodiments of the present disclosure, exemplary methods and/or materials are described below. In case of conflict, the patent specification, including definitions, will control. In addition, the materials, methods, and examples are illustrative only and are not intended to be necessarily limiting.

BRIEF DESCRIPTION OF THE SEVERAL VIEWS OF THE DRAWINGS

Some embodiments of the present disclosure are herein described, by way of example only, with reference to the accompanying drawings. With specific reference now to the drawings in detail, it is stressed that the particulars shown are by way of example, and for purposes of illustrative discussion of embodiments of the present disclosure. In this regard, the description taken with the drawings makes apparent to those skilled in the art how embodiments of the present disclosure may be practiced.

In the drawings:

FIG. 1A schematically represents a plasma generating device, according to some embodiments of the present disclosure;

FIG. 1B is a graph of typical current-voltage waveforms of atmospheric pressure plasma at low frequency (<100KHz), according to some embodiments of the present disclosure;

FIG. 1C graphs a typical high frequency (e.g., >1 MHz) current-voltage waveform of atmospheric pressure plasma, according to some embodiments of the present disclosure;

FIGs. 2A-2B schematically represent an intra-body plasma application system, illustrating a potential problem for design of a plasma generation system, addressed in some embodiments of the present disclosure;

FIG. 3A schematically illustrates a block diagram of a resonating high-voltage plasma generating system, according to some embodiments of the present disclosure;

FIG. 3B schematically illustrates a flowchart of a method of operation of a resonating high-voltage plasma generating system, according to some embodiments of the present disclosure;

FIG. 3C schematically illustrates a circuit diagram of a resonating high-voltage plasma generating system, according to some embodiments of the present disclosure;

FIG. 3D schematically represents reduction in the theoretical dependence of $C_{equiv}$ on $C_{coax}$ at gain levels $M = 1$ and $M = 2$; according to some embodiments of the present disclosure;

FIGs. 4A-4B schematically illustrate a resonating high-voltage plasma generating system, according to some embodiments of the present disclosure; and

FIGs. 5A-5B schematically illustrate a variation of a resonating high-voltage plasma generating system using a decoupling coil, according to some embodiments of the present disclosure.

## DESCRIPTION OF SPECIFIC EMBODIMENTS OF THE INVENTION

The present invention, in some embodiments thereof, relates to the field of plasma generation, and more particularly, but not exclusively, to generation and therapeutic delivery of cold plasma.

**Overview**

An aspect of some embodiments of the present disclosure relates to the design of electrical circuitry operable at high frequency generate power for plasma generation. In some embodiments of the present disclosure, an alternating electrical field is used to generate non-thermal atmospheric plasma (NTAP) via dielectric barrier discharge (DBD).

Alternation helps to overcome alternate "screening" effects caused by electrical charge mobility. During the positive cycle of the voltage (anode electrode), high-mobility electrons generated in the plasma are attracted to the electrode barrier. This produces an electrical screening effect leading swiftly to the plasma being extinguished. On the opposite cycle, when the electrode serves as cathode, these electrons become an ionization source to reignite the plasma-until attraction of positive ions to the cathode once again screens it. To achieve ongoing generation of plasma, the electrical field (proportional to the voltage applied to the electrode) is continuously alternated between these two cycle polarities. Although at fine temporal scales, plasma is emitted as discrete plasma bullets, the frequency of alternation is high enough that plasma output appears continuous.

There is, however, a potential consequence for efficiency, insofar as extinguished plasma generation must be re-ignited in each half-cycle. On a per-cycle basis, the energy required to maintain ignited plasma is considerably lower than the energy required to re-ignite it. On way to mitigate this is to reduce the time period during which mobile ions/electrodes move to strengthen screening-an effect which may be achieved in principle by increasing the cycle frequency. The frequency above which transient screening effects no longer cause intermittent extinguishing of plasma generation is referred to herein as the "transition frequency".

The transition frequency from bullet plasma to continuous plasma depends on the physical configuration of the system (e.g., electrode size, gas chamber dimensions, and/or electrode configuration), as well as the gas composition and environmental effects such as pressure and temperature. For a typical DBD plasma jet application, the transition frequency is typically about 1 MHz. Even at this frequency, screening effects sub-threshold for plasma extinction can still interfere with ionization efficiency. Accordingly, at still higher frequencies, power output that generates plasma may continue to increase, other parameters being equal.

As a result, tuning of the plasma generation frequency may be used as a way of controlling plasma power. For example, plasma generation frequency may be controllable within a range extending from somewhere near the device's transition frequency, up to about a factor often higher than that. For example, the frequency may be variable within a range from about 1 MHz up to about 10 MHz, or variable within some portion of this range.

Frequency tuning in practical electrical circuitry is subject to a variety of constraints determined and/or influenced by remaining conditions of plasma generation and performance. In some embodiments of the present disclosure, a plasma generator is operated to operate under an alternating *(e.g.,* sinusoidal) voltage with a root mean square (RMS) value of about 0.5-2 kV (for example, 1 kV RMS).

Herein, the term "continuous plasma" refers to continuity through a single high-frequency signal cycle of 100 kHz or higher. Optionally, the term refers to a train of a plurality of such cycles. However trains of such cycles may be interrupted; for example, subjected to pulse width modulation (PWM) at a frequency in a range between about 0.1 and 1 kHz *(e.g.,* 600 Hz). The duty cycle is optionally adjusted to any suitable percentage up to 100% (for example, 66%). In some embodiments, a plasma generating system may comprise PWM circuitry, e.g., PWM circuitry operable at least within such ranges.

Apart from characteristics particular to plasma generation, a generator's performance and efficiency strongly depend more generally on load characteristics and required bandwidth. In particular, a generator may be most efficient at a certain operating frequency of the circuit. For a plasma generator, this operating frequency may fall, for example, within the range of 1 MHz to 10 MHz; but other frequencies also within that range are potentially significantly less efficient.

The operating frequency may be at or near a natural resonating frequency of the circuit. Additionally or alternatively, to support a wider range of resistive and capacitive loads, a generator's design may sacrifice efficiency and performance *(e.g.,* frequency stability, distortion, and/or power loss).

In some embodiments of the present disclosure, a plasma generator is configured for use in medical field applications. The configuration includes, in particular, a spatial separation of a plasma generating unit from a power generation unit that drives it. The power generation unit may be too cumbersome to place near a treated organ and/or patient. In intra-body plasma applications where a plasma delivery probe is advanced into and/or through confined spaces, the electrical driving circuitry for the probe is simply too large to accompany the probe itself.

Utility of plasma generated outside of the body (comparatively near the driving circuitry) and delivered into it is potentially degraded by the short half-lifetime of the plasma products. Electrical characteristics of the plasma may prevent its transport by more than a few centimeters within the body.

Alternatively, a high-voltage signal is transmitted to a probe distal end, at which plasma is generated and from which the plasma is applied to target tissue. Signal transmission may be over a coaxial cable, allowing safe electrical signal transmission across a relatively long distance (e.g., a meter or more, for example, at least 1, 2, 3, 4 or 5 meters) while retaining power and signal integrity. The structure of coaxial line has potential advantages for miniaturization and flexibility when used in intra-body applications, *e.g.,* in catheter-type devices which may be advanced for distances of several centimeters *(e.g.,* 10, 50, 100 or more centimeters) beyond an opening in the body such as a natural orifice and/or a keyhole incision.

As is further described hereinbelow, use of a coaxial transmission line, though a practical solution for power transmission to a relatively remote site of plasma generation, becomes part of an interlocking system of parameters which, in some embodiments of the present disclosure, are considered constrain the transmission frequency to be near a certain resonant frequency of the transmission line (and the RC network of which it is a part) for best efficiency.

If the transmission line is part of an RC network which has a resonant frequency away from the power transmission frequency, there may be excessive losses in the transmission line itself (e.g., resulting in heating, potentially to a degree that imposes limits on operation such as reduced duty cycle), and/or inefficiencies in transmitting power into the line which increase requirements on the power supply in terms, *e.g.,* of its size, cost, and/or robustness. At resonance, inductance and capacitance can operate to cancel each other out and reduce resistive power losses.

Effects of electrical losses which produce heating in particular are potentially more significant when transmission is over a low-diameter transmission line, e.g., due to its correspondingly reduced thermal mass. In some embodiments of the present disclosure, diameter of the transmission line (e.g., less than 5 mm, 3 mm, less than 2 mm, or less than 1.5 mm) is a constraint on the minimum diameter of body cavities which can be accessed, insofar as the transmission line is used as a portion of a probe which is advanced through the body cavity to a target of plasma delivery.

A problem arises furthermore due to the high slew rate needed to provide a high voltage signal (e.g., 0.5 kV or higher RMS) at a high frequency (e.g., 1 MHz or higher). It may be difficult to cause the transmission load to follow such a slew rate, without the use of resonance driving. Resonance drive also, in some embodiments, provides a built-in power shutoff in case of accidental disconnection of or sufficiently severe damage to the transmission line, insofar as this interrupt the generation of a feedback signal which in turn entrains the frequency of power generation.

The considerations just described result in an interlocking system of engineering constraints. Here is a brief example of how constraints can interlock, which is explained in more detail below. (1) A voltage requirement at the plasma generator may be 1 kV RMS in order to achieve plasma discharge. (2) To reach 1 kV RMS from a reasonable input-side voltage of 20-33 V, the power generator's transformer gain should be in the range of about 30-50 (optionally, larger than 20, larger than 25, or larger than 30). (3) This ratio is also proportional to the square root ratio of two inductances (primary and secondary inductances) used in a transformer that can produce this gain. (4) But the output-side inductance (the secondary inductance) is constrained to a low level by (5) the same efficiency constraint that motivates selection of a high resonant frequency for delivering power to the plasma generating probe; for example a frequency of about 2 MHz. With too high an inductance, this frequency cannot be met without drastically reducing capacitance instead-but (6) this, in some embodiments, is primarily provided by the intrinsic capacitance of the coaxial cable needed to transmit power. Finally, (7), the input-side inductance (primary inductance) is constrained to be lower still than even the secondary inductance. But *this* is potentially an impractically low value. This may manifest in design pressure to abandon, for example, one or more of the desiderata of a low input voltage, a high operating frequency, or a long power transmission line.

In some embodiments of the present disclosure, the design pressure is relieved through another solution: use of a dual transformer configuration comprising a gain transformer, and a decoupling transformer (which may itself be divided into one or more stages). The gain transformer is freed to use practically reasonable inductance values to create voltage gain by the interposition of the decoupling transformer. In effect, isolation allows the output side of the gain transformer to have a low capacitance after all. Then, since there doesn't have to be any gain on the isolation transformer (or only a relatively low gain), the primary inductance of the isolation transformer can be equal to the secondary inductance, instead of having an impractically small value forced on it by a requirement to generate voltage gain. In some embodiments, decoupling effects are produced using a same inductor coil connected in parallel with both the plasma load (distally), and another inductor coil (proximally, e.g., a secondary coil of the gain transformer). This may imitate the effect of interposing a unity gain transformer, while avoiding losses due to non-ideal inductive coupling.

As potential practical results, the introduction of an isolation stage provides a potential advantage for one or more of:

- Decoupling constraints on voltage gain and on operating frequency.
- Allowing increased coaxial cable length without perturbing the ability to match other design constraints.
- Reducing frequency variation due to load impedance changes.

Details of these potential advantages are further described hereinbelow.

It is noted that electrical isolation from ground is provided, in some embodiments, by coupling of the site of plasma generation (which receives a high voltage) to its power through one or more ground-isolated transformers.

Before explaining at least one embodiment of the present disclosure in detail, it is to be understood that the present disclosure is not necessarily limited in its application to the details of construction and the arrangement of the components and/or methods set forth in the following description and/or illustrated in the drawings. Features described in the current disclosure, including features of the invention, are capable of other embodiments or of being practiced or carried out in various ways.

**Plasma Treatment Device**

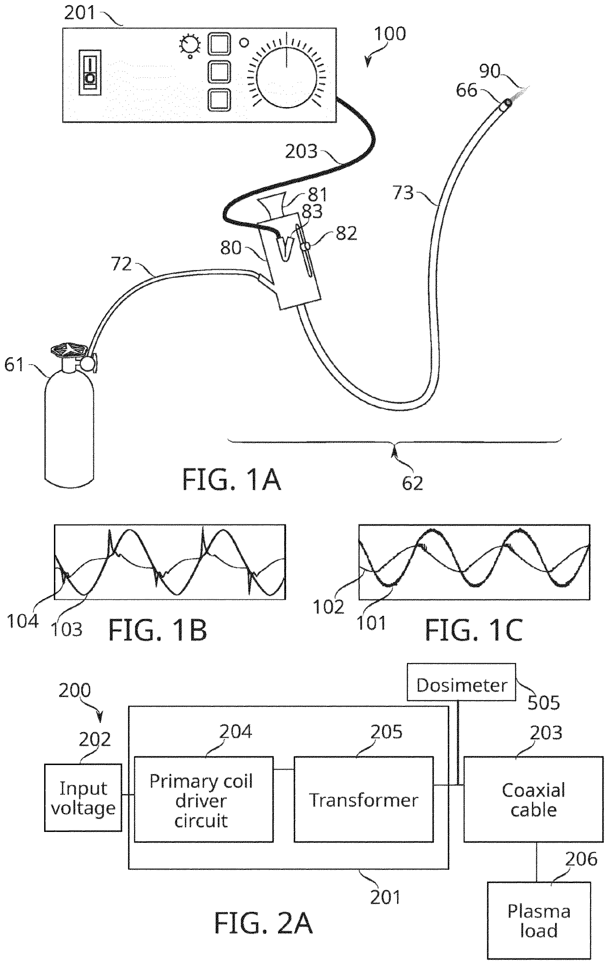

Reference is now made to *Figure 1A,* which schematically represents a plasma generating device **100,** according to some embodiments of the present disclosure.

Plasma generating device **100,** in some embodiments, comprises a high voltage power generator **201** and an ionization gas supply **61** interconnected to a plasma probe assembly **62.** High voltage power generator **201** supplies ionizing voltage to plasma probe assembly **62** via cable **203** (which may be, for example, a coaxial cable, or another electrical conduit with controlled impedance). Ionizing gas supply **61** supplies an ionization gas to plasma probe assembly **62** via tubing **72.** The supplied gas may comprise, for example, one or more noble gases such as neon, argon, or helium; and/or other gas(es) suitable for ionization into a plasma plume 90. Optionally, cable **203** and tubing **72** are integrated into a single cabling unit which connects with plasma probe assembly **62.** Optionally, high voltage power generator **201** and ionization gas supply **61** are integrally housed.

Plasma probe assembly **62,** optionally comprises a handle **80.** Handle **80** is optionally provided with controls **81, 82** for controlling actuation of probe conduit **73** and/or plasma delivery tip **66,** for controlling functions of power generator **201,** and/or for controlling ionization gas delivery from gas supply **61.** Optionally, plasma probe assembly **62** physically integrates power functions and gas delivery functions into probe conduit without use of a dedicated handle. In some embodiments, probe conduit **73** includes both a lumen for delivery of ionization gas, and a high voltage transmission line *(e.g.,* a continuation of cable **203** and tubing **72).** In some embodiments, probe conduit comprises a plurality of lumens, *e.g.,* a lumen attached to gas supply **61** which delivers of ionization gas, and a lumen which scavenges (removes) of ionization gas, optionally under suction. In some embodiments, any one or more of the lumens of probe conduit **73** is optionally used as a working channel, by insertion of a tool. Handle **80,** in some embodiments, comprises one or more ports **83** for introduction of such tools into a lumen of probe conduit **73.**

In some embodiments of the present invention, probe conduit **73** and plasma delivery tip **66** are sized and otherwise configured *(e.g.,* safety-configured) for the delivery of non-thermal plasma to an intrabody location. The intrabody location may be remotely located relative to a point of insertion, *e.g.,* distant from a point of insertion into the body by 25 cm or more; for example, 80 cm, 100 cm, or 120 cm. Insofar as plasma tip **60** comprises the site of plasma generation (as it does in some embodiments), the device must supply ionization-level voltage *(e.g.,* a voltage of 1 kV or more) to the tip **60.** This length may place a constraint on the minimum capacitance of cable **203.**

In some embodiments, probe conduit 73 (including both a lumen for delivery of ionization gas and high voltage transmission line) has an overall diameter of less than 12 mm, less than 10 mm, less than 8 mm, or less than 5 mm. The narrower the overall diameter, the narrower the minimum-sized body lumen that the probe conduit can traverse in the manner of a catheter; for example, a ureter, blood vessel, or other subcutaneous access-way to a targeted treatment site.

Reference is now made to *Figure 1B,* which is a graph of typical current-voltage waveforms of atmospheric pressure plasma at low frequency (<100KHz). Reference is also made to *Figure 1C,* which graphs a typical high frequency *(e.g.,* > 1 MHz) current-voltage waveform of atmospheric pressure plasma.

While the excitation voltage **103, 101** is sinusoidal in both cases, it may be seen in *Figure 1B* that the current **104** spikes as plasma bullet is ignited on each half-cycle. In the higher frequency example of *Figure 1C,* the current waveform **102** more closely tracks the input voltage (with a phase shift) as continuous plasma generation maintains uniform resistance in plasma plume.

## Plasma High-Voltage Generator

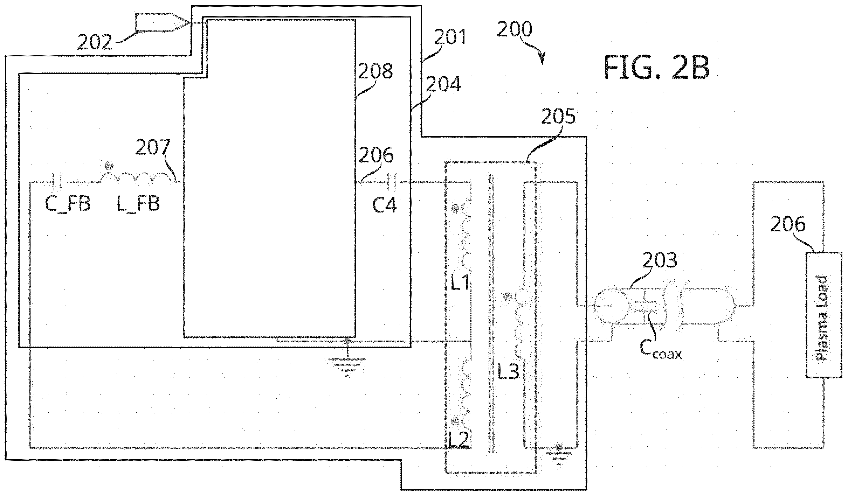

Reference is now made to *Figures 2A-2B,* which schematically represent an intra-body plasma application system **200,** illustrating a potential problem for design of a plasma generation system, addressed in some embodiments of the present disclosure. *Figure 2A* is a schematic block diagram of system **200,** while *Figure 2B* represents a component-level implementation of the schematic block diagram of *Figure 2A.*

In the example shown, generator **201** and coaxial cable **203** operate together to generate a high-voltage driving signal for the plasma gun. Generator **201** comprises a primary coil driver circuit **204** comprising amplifier **208,** activated by input voltage **202,** and driving the primary coil of transformer **205.** Coaxial cable **203,** in some embodiments, comprises a flexible electrical transmission line interconnected with the secondary coil of transformer **205,** so as to conduct a high voltage to a plasma generating site near its terminal end, whereat plasma is generated which constitutes the plasma load **206.**

To generate plasma, an ionizing gas is also conducted to the plasma generating site, optionally through a conduit which extends alongside coaxial cable **203** to reach the plasma generating site. In some embodiments, the coaxial cable **203** is inserted within a body to be treated (e.g., inserted along the lumen of a body cavity) while the high voltage delivered, along with ionizing gas, to generate plasma. The voltage high-frequency alternating; *e.g.,* at a frequency of several kilohertz, for example, 100 kHz or higher. It is noted that dosimetry of the plasma produced is optionally achieved by one or more of several arrangements and/or methods. For example, the signal *(e.g.,* its power, frequency, and/or waveform) used to generate plasma may be monitored by dosimetry circuitry *(e.g.,* circuitry in communication with optional dosimeter **505)** arranged to make measurements of the signal just before it enters coaxial cable **203,** and/or at another location in circuitry the overall system. Optionally, the plasma itself is monitored, *e.g.,* optically or electrically, by sensors near to the site of plasma generation (plasma load 206), communicating with dosimeter **505.**

The implementation of *Figure 2B* indicates the capacitance $C_{coax}$ of coaxial cable **203,** which, in some embodiments, adds significant capacitance to the overall load driven by generator **201** via primary coil driver circuit **204.** This capacitance is a general property to be expected of any type of transmission line.

With increasing targeted frequency of operation, this added capacitance eventually becomes limiting on the bandwidth that generator *201* can achieve for a given voltage amplitude. At relatively low frequencies of several kHz, the added capacitance $C_{coax}$ is insignificant for realistic cable lengths of 2-3 m.

However, to increase the frequency into the MHz range, it is a potential advantage to use a resonating generator configuration, such as is shown in *Figure 2B.* The resonating generator relies on the cable capacitance to form (along with transformer *205)* an 'LC'-type resonator circuit which can be tailored to a specific resonance frequency. This generated frequency, along with the high voltage provided from the transformer high-side (the secondary coil of the transformer), are fed directly to the plasma generating portion.

It should be noted that this power signal generation approach is distinct in character from an approach which generates a power signal of a frequency which is merely selected to match the time constant of the load. The matching in that case is needed for efficient transmission of a signal already generated. Integration of the load and its electrical characteristics into the generator circuit itself has potential disadvantages for flexibility of power generation parameters. For example, frequency selection adjustment in a standard power supply should match load impedance to get efficient transmission into the load, but the frequency of the power supply signal *itself* may be selectable relatively freely without preventing its generation. When the load becomes part of the signal generating circuit, adjustment of frequency outside of a limited range means adjusting electrical characteristics of the load and driver circuits in concert. If adjustment is not concerted, the signal generating circuit may simply cease to generate a signal at all.

In some embodiments of the present disclosure, matching performs a function over and above achieving efficient transmission of a power signal; it also affects generation of the power signal itself. The frequency of that power signal is intrinsically (that is, as a matter of proximate cause, not just as a matter of predetermined selection) generated as a function of the load characteristics. Change the load, and the frequency changes too. This is not to say that the power supply will generate efficiently at *any* frequency selected by the resonance of the LC circuit comprising $C_{coax}$ and *L3;* it must operate within its own limits. Change the load characteristics *too* much, and power signal generation itself is impaired. In the entire absence of load-side resonance, power signal generation stops, due to extreme network mismatch.

In effect, the transmission line capacitance acts as a key component of the timing signal providing portion of the power signal generator, in such a fashion that either the transmission line inherently can accept the frequency of the power signal being generated (having generated the frequency, it can also accept it)-or else, there is no effective power signal to accept. In effect, the load side of the circuit entrains the oscillation of the driver circuit, in distinction to merely being selected to match its oscillating frequency.

Restated briefly, the capacitance $C_{coax}$ together with the secondary coil inductance *L3* are used as primary parameters determining the time constant with which not only they, but also the driving circuit *204* on the primary side of coil *205* oscillate. However, at a sufficiently high frequency, this theoretical circuit becomes practically difficult to realize; for example, because the voltage gain requirements force the selection of an unrealistically low inductance onto the primary coil (comprising inductors L1, L2) of transformer **205.**

In the simplified electrical schematic of *Figure 2B,* primary coil driver circuit **204** is fed by an input voltage source **202,** e.g., a DC voltage measuring up to 100 V. A transformer unit **205** is connected between driver circuit **204** and the transmission line (coaxial cable **203**). It comprises 3 coupled inductors: primary coil **L1,** feedback coil **L2** and secondary coil **L3.**

Amplifier **208** output **206** is connected via decoupling capacitor **C4** to the primary coil **L1,** while the feedback to amplifier **208** at opposing polarity is connected via compensation network **C_FB** and **L_FB** to the input **207.** Matching the time constant of the LC network comprising **L3** and $\mathbf{C_{coax}}$ by the feedback network comprising **L2, L_FB** and **C_FB** results in sinusoidal resonance at a frequency of:

$$f = \frac{1}{2\pi\sqrt{L_3 \cdot C_{coax}}}$$

Considering a typical use-case, there may be, for example, a 2 MHz resonance targeted. A typical capacitance of 2 m of coaxial cable may be, for example, about 200 pF. With these as givens, the secondary coil inductance may be selected to measure 31 $\mu$H.

In some embodiments, capacitance of the transmission line (the transmission line comprising e.g., coaxial cable, twisted pair cable, or another conductor configuration) is at least 50 pF, 100 pF, 150 pF, or 200 pF. In some embodiments, the length of the transmission line is at least 50 cm, 100 cm, 150 cm, 200 cm. Secondary could inductance is selected accordingly.

As a further design parameter, a modest working high-voltage of 1 kV RMS may be assigned at the plasma load **206,** but higher working voltages may be desired, for example, at least 1.5 kV RMS or at least 2 kV RMS.

For reasonable input voltages the transformer **205** will have a gain ratio in the range of about 30-50. Insofar as the gain ratio is in proportion to the square root of the inductance ratio, this places a constraint on the primary coil inductance to be in the range of approximately 0.01-0.03 $\mu$H:

$$\text{Transformer Gain} = \sqrt{\frac{L_{Secondary(3)}}{L_{Primary(1)}}}$$

Use of such a low primary-side inductance in a transformer design for use with such high voltages is impractical. Physical constraints ordinarily lead to single coil inductors of a few nH having typical sizes of only a few millimeters *(e.g.,* 4 mm or less) in their largest dimension. A corresponding transformer, even if somehow constructed, would have to handle huge current loads at the target voltages (0.01 $\mu$H is equivalent to about 0.1 $\Omega$ at 2 MHz), resulting in transformer saturation, among other parasitic effects such as the skin effect. There are also concerns with heating and electrical insulation. Increasing input voltage (which allows reducing gain while maintaining the targeted output voltage), significantly decreases generator efficiency (also resulting in heat), and beyond a certain point also becomes impractical.

At more moderate frequencies, constraints relax enough that transformer **205** may be of practical use. For example, for a plasma generator unit driven by 1 kV RMS at 1 MHz into a 100 pF cable, the following driving circuitry can be used: a 100 $\mu$H secondary side inductance, a 1 $\mu$H primary side inductance (leading to a gain of about 10), and an input voltage of 90 V. The transformer's feedback coil inductance is typically of less importance but ordinarily is about the same as the inductance of the primary side coil.

If, in the above example, the capacitance of coaxial cable **203** is modified so that $\mathbf{C_{coax}}$ is 100 pF (other components remaining equal), there is expected to be a resulting sharp rise of frequency, reduction in efficiency, and potentially even complete loss of resonance if further decrease in capacitance is performed. Similarly, increasing the cable capacitance *(e.g.,* by increasing its length) will result in decreased frequency and loss of efficiency. In either case, the loss in efficiency is due to the introduction of a mismatch between the feedback **(C_FB, L_FB** and **L2)** LC network and the primary (**L3** and $\mathbf{C_{coax}}$) LC network.

*High-Voltage Generator with Dual- Transformer stages*

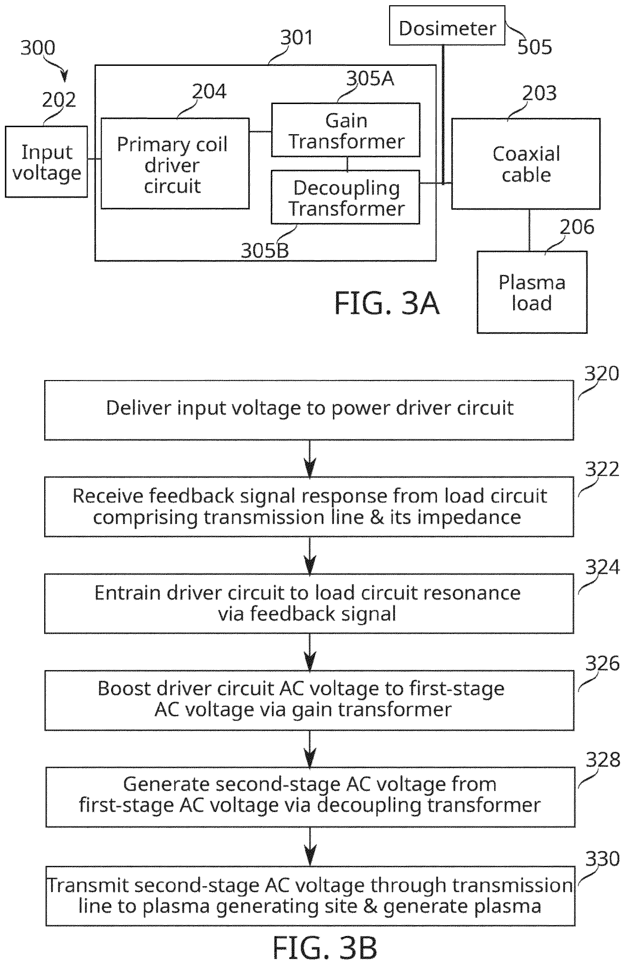

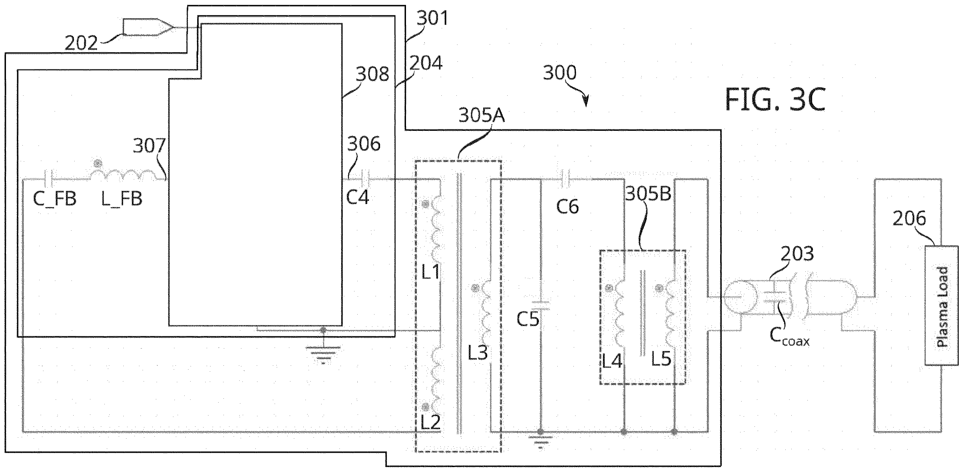

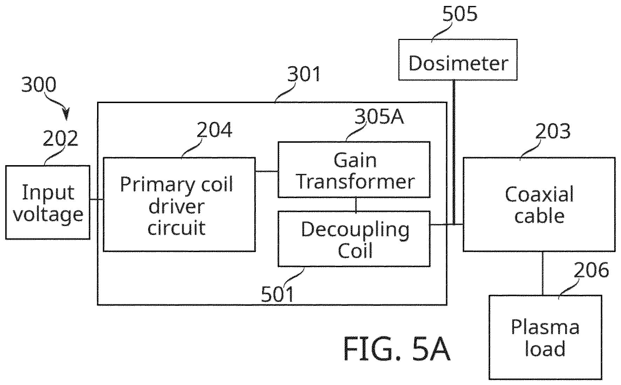

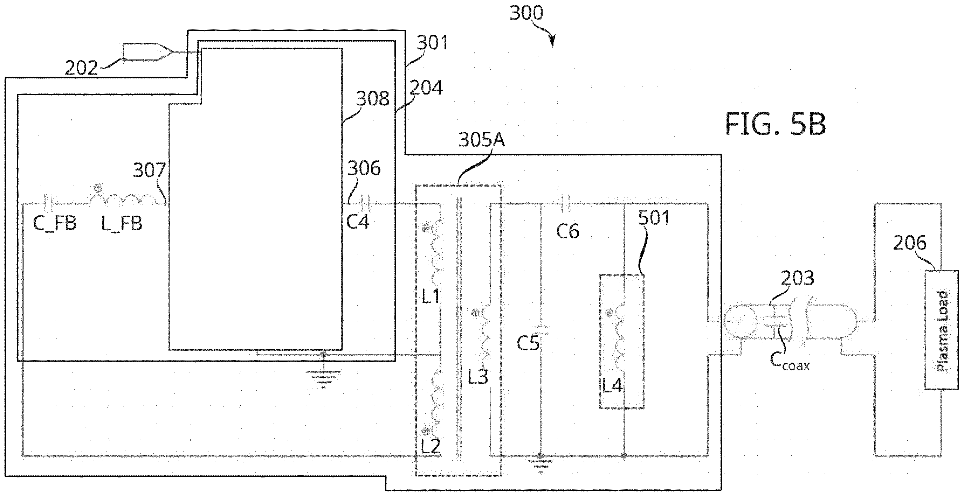

Reference is now made to *Figures 3A* and *3C,* which schematically illustrate a resonating high-voltage plasma generating system **300,** according to some embodiments of the present disclosure. Reference is also made to *Figures 5A* and *5B,* which schematically illustrate a variation of a resonating high-voltage plasma generating system **300** using a decoupling coil **501,** according to some embodiments of the present disclosure. Compared to the block diagram of *Figure 2A,* generator **301** of *Figures 3A* and *5A* divides transformer **205** into a two-stage transformer: a gain transformer **305A,** and a decoupling transformer **305B** or decoupling coil **501.** Descriptions with respect to *Figures 3A* and *3C* also apply to the embodiments of *Figures 5A* and *5B,* changed as appropriate to accommodate the substitution of decoupling coil **501** for decoupling transformer **305B.** This substitution is related to further hereinbelow.

In some embodiments, this allows driving a relatively high-capacitance load while retaining potential advantages of a resonating architecture to achieve a high frequency and potentially true sinusoidal output while generating plasma.

Considering driver circuit **204** (comprising amplifier **308**, output **306**, and feedback input **307**) in combination with gain transformer **305A,** basic resonance is achieved by matching the time constant of the **L3** and **C5** LC network with the feedback LC network comprising **(C_FB, L_FB,** and **L2**). **C5** can be selected as a small value *(e.g.,* within a factor of ten of 1-5 pF). This allows the corresponding secondary coil inductance **L3** to be substantially higher. This also allows the primary and/or feedback coil inductances *(e.g.,* **L1** and/or **L2**) to be substantially higher, potentially entering into a practical range that allows a gain which was otherwise not available at frequencies of, for example, 1-2 MHz. In some embodiments, primary coil **L1** has an inductance in the range of about 1-5 $\mu$H, secondary coil **L3** has an inductance in the range of about 1000-5000 $\mu$H, and feedback coil **L2** has an inductance in the range of about 0.1-10 $\mu$H.

The remainder of the circuit delivers the high frequency, high voltages signal to the load. Capacitor **C6** decouples the generated signal. It may measure, for example, within a range from several tenths of a nanoFarad up to hundreds of nF *(e.g.,* in a range of 0.1-900 nF; for example, 0.5-2 nF, 2-10 nF, 0.1-10 nF, 5-20 nF, 20-100 nF, 50-500 nF, or another capacitance range). Relatively lower capacitances have the potential advantage of increasing the rates of system response, for example in aspects such as oscillation frequency and rate of voltage stabilization (meaning-voltage reaches its driven values sooner, since there is less capacitance being charged). Relatively higher capacitances have the potential advantage of reducing general instability characteristics, such as a tendency to overshoot the intended voltage, and potentially to not always reach the same voltage *(e.g.,* instability cycle-to-cycle, and/or unstable mode of operation).

Beyond it, an additional decoupling transformer **305B** is used, having a primary side inductance **L4** and secondary side inductance **L5.** Optionally, this transformer is configured to unity gain **(L4 = L5,** optionally chosen to a value within about a factor of ten of 100 $\mu$H, for example, a value within the range of 20-200 $\mu$H). Alternatively, this transformer is used as a second stage gain. For example, by setting **L4** to a value in the range of about 5-20 $\mu$H and **L5** to a value in the range of about 20-80 $\mu$H; *e.g.,* such that an effective second stage gain of 1-4 (for example, 2) is achieved. Finally, the LC network comprising **L5** and $C_{coax}$ is designed to match the resonance frequency set by the driver circuit **204** and the gain transformer.

Dosimetry is optionally performed using optional dosimeter **505;** for example, using one or more of the implementations described in relation to *Figure 2A.*

In some embodiments, the role of decoupling transformer **305B** *(Figures 3A* and *3C)* is performed by a single decoupling coil **501** *(Figures 5A* and *5B),* connected in electrical parallel with the plasma load **206.** It may be understood that in embodiments wherein a decoupling transformer **305B** provides unity gain, there is a potential loss of ideal behavior insofar as coupling between the primary side and secondary side is non-ideal. This potentially includes efficiency losses. However, decoupling coil **501** is used, in some embodiments, as a substituted equivalent to a unity gain decoupling transformer **305B;** *i.e.,* equivalent insofar as the function of providing inductance which affects the circuit's resonant frequency is concerned. This substitution provides the potential advantage of avoiding transformer losses, while maintaining certain of the potential advantages described in the following discussion *(i.e.,* those which do not rely on having a gain other than one).

The embodiments of the two pairs of figures *(Figures 3A* and *3C* as one pair, and *Figures 5A* and *5B* as the other pair) may be characterized in common as providing decoupling of the impedance of the plasma probe from the impedance characteristics of the gain transformer. More particularly, they decouple the constraints of probe lead capacitance (coaxial cable capacitance) from constraints of gain transformer design which affect characteristics such as the operating voltage, carrier frequency, and/or efficiency of the system. They may be characterized in common as each including an inductance connected between the conductors of coaxial cable **203** and/or in parallel with plasma load **206;** and as including an inductance connected in parallel with a secondary coil of transform **305A** (having secondary coil inductance **L3)** and/or in parallel with capacitance **C5.** In the case of the embodiments of *Figures 5A-5B,* each such defined "inductance connected in parallel" is embodied in the same inductance provided by the same inductive component (e.g., an inductor coil), while in the case of the embodiments of *Figures 3A* and *3C,* there are two separately provided inductive components *(e.g.,* two inductor coils of a transformer). Accordingly, there is provided in either case at least one decoupling inductor, providing inductance including inductance connected in parallel to the secondary coil, and inductance connected in parallel to the plasma generating site.

While it is potentially convenient to provide each inductive component as a single inductive coil, there is no inherent obstacle to providing the inductive components in other configurations of inductive sub-components arranged in electrical series and/or electrical parallel as may be suitable to provide a targeted effective inductance influencing the rest of the circuit. Nor is there a particular impediment to partial sharing and partial separation of the separately defined inductances, which is optionally provided in some embodiments of the present disclosure. In some embodiments, some non-shared portion of the differently defined inductances may optionally be provided in the form of a transformer having a non-unity gain.

There are some electrical differences due to the substitution; however, these may be reasonably understood as not impairing or distorting the essential function of the circuit. For example, it may be noted that in *Figure 3C,* only one side of each of the coils providing inductances L4, L1 is conductively connected, while the embodiment using decoupling coil **501** is, effectively, conductively connected on both sides. However, the use of unity gain means that there is (again, ideally) no voltage potential difference on either side of the coils in any case, so that conductively interconnecting them does not affect their behavior. Differences in voltage and/or phase which may occur in the non-ideal case for embodiments using a decoupling transformer **305B** are not particularly relied on or essential to device function.

There may also be differences in single coil current carried. Optionally decoupling coil **501** is designed *(e.g.,* with a heavier conductor) to withstand higher currents *(e.g.,* 2x higher) than either of the individual coils of decoupling transformer **305B** carries alone.

At least three substantial potential advantages are identifiable which may achieved by one or more of these configurations compared to traditional resonant transformer configuration.

First, coupling between frequency and gain is loosened by adding an additional design degree of freedom. In the design of *Figures 2A-2B:*

$$f = \frac{1}{2\pi \cdot \text{Gain} \cdot \sqrt{L_1} \cdot \sqrt{C_{coax}}} = \frac{K}{\text{Gain} \cdot \sqrt{L_1}}$$

Where the capacitance $\mathbf{C_{coax}}$ ends up being absorbed into the design requirement-fixed constant *K,* insofar as it is constrained to be a relatively large value. This yields the already described inverse relationship between frequency *f* and the inductance **L1** and the design gain. Increasing the frequency requires reducing either or both of **L1** and the gain to levels with potentially impractical consequences for construction and/or operation of the device.

Using a second transformer stage introduces the option to use **C5** as a compensation capacitor (an additional degree of design freedom):

$$f = \frac{1}{2\pi \cdot \text{Gain} \cdot \sqrt{L_1} \cdot \sqrt{C_{coax}} \cdot \sqrt{C_5}} = \frac{K}{\text{Gain} \cdot \sqrt{L_1} \cdot \sqrt{C_5}}$$

Second, limitations on coaxial cable length are relaxed. As just mentioned, $\mathbf{C_{coax}}$ may be considered as having a lower limit imposed by design requirements to have a long, thin, flexible cable to allow reaching remote targets to which plasma is applied. It is a potential advantage, in some embodiments of the present disclosure, to lengthen coaxial cable **203** beyond the length that results in this lower limit. The introduction of **C5** as a design degree of freedom also allows this. While the minimum practical value of transformer inductance **L5** remains a limitation for any given selected target resonant frequency *f*, it can still be much smaller in the decoupling transformer **305B** than in a gain transformer **305A,** since a requirement that the primary inductance **L4** be much smaller still has been removed.

Third, variation in frequency due to changes in load impedance may be reduced, at least if the second stage gain is selected to be greater than one. Impedance could change, for example, a result damage. It is a potential advantage to reduce sensitivity to minor fluctuations, while still retaining the auto-attenuating effects of large impedance changes. Even for a fixed load impedance, manufacturing tolerances of several percent *(e.g.,* 10%) may apply to key electrical properties of components, potentially including the capacitance of a transmission line. It is a potential advantage to reduce sensitivity of a resonance driver circuit to variation in, *e.g.,* transmission line capacitance, while also retaining advantages of a resonance circuit, for example self-stopping of high frequency signal generation should the transmission line become disconnected.

In a single-stage resonant generator, the capacitance of the transmission line directly relates to frequency as $f \propto (C_{coax})^{-0.5}$. Adding a second stage adds significant complexity to this relation. The transmission line and second stage together may be "seen" as an equivalent capacitance, as far as effect on frequency are concerned, such that $f \propto$

$(C_{equiv})^{-0.5}$. A theoretical dependence of $C_{equiv}$ on $C_{coax}$ may then in turn be expressed as $C_{equiv.} \propto \frac{C_{coax}}{M^2}$, where the effective gain of the second stage is $M$.

From this, it may be understood that setting a gain $M > 1$ reduces variation in oscillation frequency due to (absolute) changes in load impedance. For simplicity, ohmic components present in a practical system were omitted in the above relation *(e.g., electrical series resistance)*. Considering these, sensitivity to load impedance may increase somewhat.

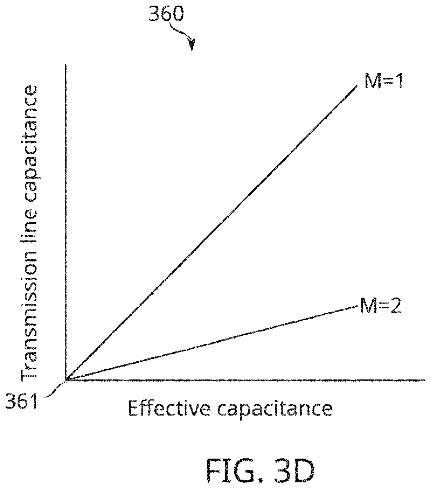

Brief reference is now made to *Figure 3D,* showing a graph 360, which schematically represents reduction in the theoretical dependence of $C_{equiv}$ on $C_{coax}$ at gain levels $M = 1$ and $M = 2$. At the origin **361** is a zero transmission line capacitance; the effective capacitance is a baseline value, not necessarily zero.

With increasing gain *M,* dependency of effective capacitance on transmission line capacitance reduces. Details of this relationship will vary in practical circuits, *e.g.,* according to the presence of other impedances such as parasitic impedances.

In system **300,** if the coaxial cable **203** is disconnected from the circuit, there remains a resonance circuit on the primary side of the decoupling transformer. However, disconnection will also change how the decoupling transformer contributes to that resonance circuit, leading in any case to a likely loss of high-frequency power generation.

When the coaxial cable **203** is re-attached, the effective resonance of the two sides of the decoupling transformer together is still part of the load side of the circuit. This is true in the functional sense that if the circuits on either side of the decoupling transformer don't allow resonance at a mutually compatible frequency, they can't provide the needed frequency that allows the driver circuit **204** to perform its switching. The true (power dissipating) load circuit is still acting to instruct the driver circuit, albeit through and in co-operation with the circuit driving the primary inductance of the decoupling transformer.

As noted above, that influence can diminish with increasing gain on the decoupling transformer. However, even in embodiments where a gain higher than one is used, the effect of an impedance mismatch *(e.g.,* due to damage to the coaxial cable **203)** is characteristic. If the effect on the resonance of the primary side of the decoupling transformer manages (despite the gain) to be large enough, it leads to the same failure mode as before: loss of load resonance amplitude, loss of driver circuit entrainment, and ultimately reduction of power or potentially failure to generate power. To the extent that the impedance mismatch is *not* large enough to seriously reduce resonance of the primary side of the decoupling transform, then the problem is halted too: the driver circuit **204** does not see it. The state of the load circuit remains a failsafe.

Reference is now made to *Figure 3B,* which schematically represents a flowchart of a method of operation of a resonating high-voltage plasma generating system according to some embodiments of the present disclosure.

At block **320,** in some embodiments, input voltage **202** is delivered to a power driver circuit *(e.g.,* comprising block **204** of *Figure 3A).*

*Figure 3B* presents the operations of blocks **322-330** are presented in an order *(e.g.,* indicated by the arrows) suitable for descriptive purposes of their mutual dependencies; however it should be understood that operations in an actual device are casually interrelated such that they may all develop simultaneously and/or at least partially in reaction to each other, as aspects of the operation of the circuitry as a whole. For example, the feedback signal mentioned at block **322** is itself established, in some embodiments, as a result of electrical field interactions occurring at the gain transformer, and mentioned in block **326,** which in turn are affected by the generation (block **328)** and transmission (block **330)** of second-stage AC voltages. Effects of the operations of block **330** may be considered, accordingly, as leading back upon the operations of block **322,** in the sense of indicating mutual dependency of occurrences in the circuit. Thus, one may have a condition wherein without the feedback of block **322** there is no AC voltage generated and transmitted at blocks **328, 330,** but also it may be said that without that AC voltage, there is no feedback.

At block **322,** in some embodiments, and via at least gain transformer **305A,** a feedback signal generated in response to activation in turn by the power driver circuit is received from a load circuit comprising at least a transmission line *(e.g.,* coaxial cable **203).**

At block **324,** in some embodiments, the feedback signal entrains the driver circuit to oscillate, the oscillation being tuned (through the feedback signal) to the resonant frequency of the load circuit.

At block **326,** in some embodiments, an AC voltage generated by the entrained driver circuit is boosted to a higher voltage by inductive coupling at a gain transformer **305A.** The higher voltage is present on the secondary (output) side of the gain transformer **305A** in a first stage of the load-circuit side of the overall power generating circuit.

At block **328,** in some embodiments, the AC voltage on the first-stage (primary/input) side of decoupling transformer **305B** induces a voltage on the second-stage (secondary/output) side of decoupling transformer **305B.** In some embodiments, gain at decoupling transformer **305B** is equal to or lower than the gain on the gain transform **305A.** Optionally gain at decoupling transformer **305B** is about 1 *(e.g.* no gain). Optionally, decoupling transformer **305B** is replaced by a decoupling coil **501** or another configuration of decoupling inductances; for example as described in relation to *Figures 5A-5B.* Optionally, the gain of gain transformer **305A** is smaller than gain at gain transformer **305B** by a factor of at least 1.5, 2, 3, 4, 5 or more.

At block **330,** in some embodiments, the second-stage voltage is transmitted along a transmission line *(e.g., coaxial cable 203 or a cable of another design, for example, twisted pair),* reaches a plasma generating site, and provides power used to generate plasma.

**High-Voltage Generator with Multiple-Transformer Stages**

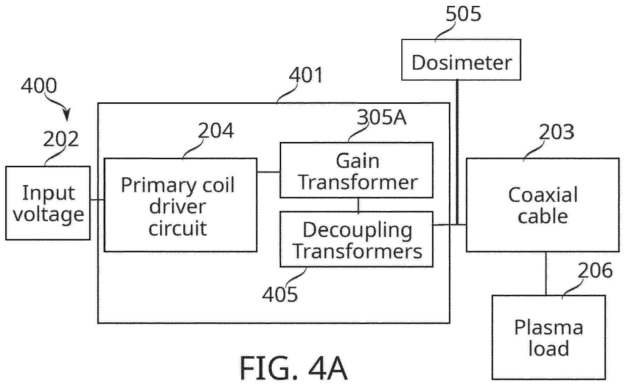

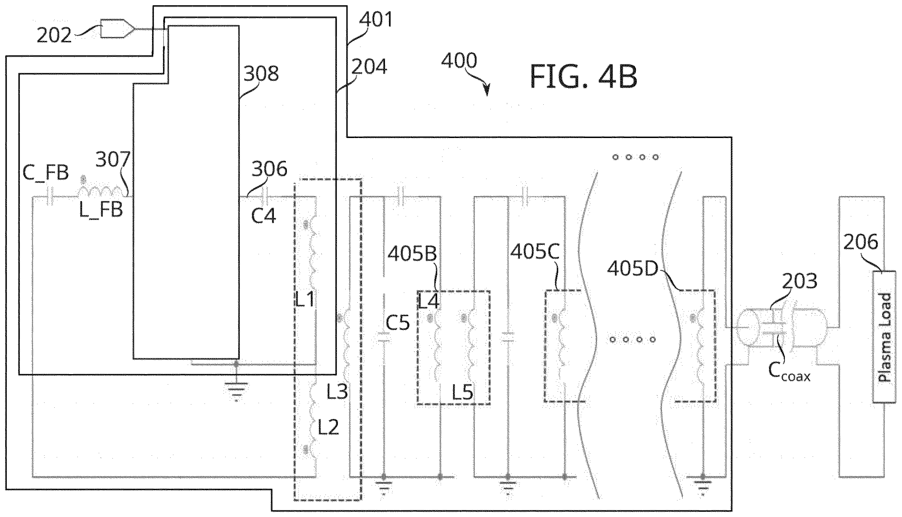

Reference is now made to *Figures 4A-4B,* which schematically illustrate a resonating high-voltage plasma generating system **400,** according to some embodiments of the present disclosure. Compared to the block diagram of *Figure 3A,* generator **401** of *Figure 4A* divides decoupling transformer **305B** into a plurality of *n* decoupling transformers **405** where $n \geq 2$. The plurality of decoupling transformers are represented in *Figure 4B* as sub-stages **405B, 405C,** and **405D,** driven from driver circuit **204** (which comprises amplifier **308** having feedback input **307** and output **306**). Not all components are shown for sub-stages beyond **405B,** but they may be understood as repeating the unit of sub-stage **405B** with the connectivity shown. Sub-stage **405C** may be understood as representing one or more "intermediate" sub-stages. Dosimetry is optionally performed using optional dosimeter **505;** for example, using one or more of the implementations described in relation to *Figure 2A.*

Division of the decoupling transformer **305B** into a plurality of decoupling transformers **405** generalizes on the dual-transformer architecture of *Figures 3A* and *3C.* Although more complex, the multiple stage design has the potential advantage of distributing voltage gain, if such is required, beyond what may be practical in a two transformer design while maintaining a high operating (resonant) frequency.

Another potential advantage is reduced sensitivity to variations of the load impedance, generalizing to:

$$C_{equiv.} \propto \frac{C_{coax}}{M_1{}^2 M_2{}^2 \ldots M_n{}^2} = \frac{C_{coax}}{\prod_1^n M_n{}^2}$$

Where: $M_n$ is the gain of the $n^{th}$ transformer stage. Impedance matching is preferably still attended to, however, to prevent signal reflections and harmonic distortions. It should be understood that each additional stage reduces overall efficiency, particularly in practical systems including resistive losses and parasitic capacitances.

The method of *Figure 3B* also applies, in some embodiments, to the operation of the device of *Figures 4A-4B,* changed as necessary to account for additional stages. For example, it applies with the modification that the operations of block **328** are duplicated as necessary among a plurality of decoupling stages until the last decoupling stage is reached, at which said last stage transmission of the final AC voltage occurs through the transmission line to the plasma generating site to generate plasma.

In some embodiments, the transmission line (cable **203)** itself is part of one or more of the intermediate stages, with one or more decoupling transformers located along the transmission line. This includes the option of dividing cable **203** into inductively coupled segments.

**General**

As used herein with reference to quantity or value, the term "about" means "within $\pm 10\%$ of".

The terms "comprises", "comprising", "includes", "including", "having" and their conjugates mean: "including but not limited to".

The term "consisting of" means: "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

The words "example" and "exemplary" are used herein to mean "serving as an example, instance or illustration". Any embodiment described as an "example" or "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the present disclosure may include a plurality of "optional" features except insofar as such features conflict.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

Throughout this application, embodiments may be presented with reference to a range format. It should be understood that the description in range format is merely for convenience and brevity and should not be construed as an inflexible limitation on the scope of descriptions of the present disclosure. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", *etc.;* as well as individual numbers within that range, for example, 1, 2, 3, 4, 5, and 6. This applies regardless of the breadth of the range.

Whenever a numerical range is indicated herein (for example "10-15", "10 to 15", or any pair of numbers linked by these another such range indication), it is meant to include any number (fractional or integral) within the indicated range limits, including the range limits, unless the context clearly dictates otherwise. The phrases "range/ranging/ranges between" a first indicate number and a second indicate number and "range/ranging/ranges from" a first indicate number "to", "up to", "until" or "through" (or another such range-indicating term) a second indicate number are used herein interchangeably and are meant to include the first and second indicated numbers and all the fractional and integral numbers therebetween.

Although descriptions of the present disclosure are provided in conjunction with specific embodiments, it is evident that many alternatives, modifications and variations will be apparent to those skilled in the art. Accordingly, it is intended to embrace all such alternatives, modifications and variations that fall within the spirit and broad scope of the appended claims.

It is appreciated that certain features which are, for clarity, described in the present disclosure in the context of separate embodiments, may also be provided in combination in a single embodiment. Conversely, various features, which are, for brevity, described in the context of a single embodiment, may also be provided separately or in any suitable subcombination or as suitable in any other described embodiment of the present disclosure. Certain features described in the context of various embodiments are not to be considered essential features of those embodiments, unless the embodiment is inoperative without those elements.

It is the intent of the applicant(s) that all publications, patents and patent applications referred to in this specification are to be incorporated in their entirety by reference into the specification, to the same extent as if each individual publication, patent or patent application was specifically and individually noted when referenced that it is to be incorporated herein by reference. In addition, citation or identification of any reference in this application shall not be construed as an admission that such reference is available as prior art to the present disclosure. To the extent that section headings are used, they should not be construed as necessarily limiting. In addition, any priority document(s) of this application is/are hereby incorporated herein by reference in its/their entirety.

## Claims

1. A non-thermal plasma generator comprising:

   a gain transformer comprising a primary coil and a secondary coil;
   a driver circuit electrically connected to drive a current through the primary coil;
   a load circuit having a distal end comprising a plasma generating site generating non-thermal plasma, and a proximal end coupled to the secondary coil of the gain transformer;
   wherein the load circuit comprises at least one decoupling inductor, providing inductance including inductance connected in parallel to the secondary coil, and inductance connected in parallel to the plasma generating site.

2. The non-thermal plasma generator of claim 1, wherein the load circuit has an impedance determining a frequency of oscillation of the load circuit in response to a current generated in the secondary coil.

3. The non-thermal plasma generator of claim 1 or 2, wherein the load circuit entrains oscillation of the driver circuit.

4. The non-thermal plasma generator of claim 3, wherein oscillation of the driver circuit is entrained via feedback from the gain transformer, optionally, wherein the feedback from the gain transformer is provided by a feedback winding of the gain transformer, and wherein the feedback winding has an inductance in the range of about 1-10 µH.

5. The non-thermal plasma generator of any of claims 2 to 4, wherein the frequency of oscillation of the load circuit is sufficiently high that plasma generation at the plasma generating site does not extinguish during at least a full oscillation cycle.

6. The non-thermal plasma generator of any of claims 2 to 5, comprising pulse modulation circuitry operable to modulate the frequency of oscillation at a lower frequency within the range of 0.1-1 KHz.

7. The non-thermal plasma generator of any of claims 1 to 6, wherein the gain transformer provides a gain of at least 20.

8. The non-thermal plasma generator of any of claims 1 to 7, wherein the at least one decoupling inductor comprises at least one decoupling transformer, providing in aggregate a gain no larger than 1 or in aggregate a gain smaller than the gain provided by the gain transformer by a factor of at least 2.

9. The non-thermal plasma generator of any of claims 1 to 8, wherein the at least one decoupling inductor comprises an inductor coil connected both in parallel to the secondary coil, and in parallel to the plasma generating site.

10. The non-thermal plasma generator of any of claims 2 to 9, wherein the driver circuit ceases oscillation when the plasma generating site is disconnected from the load circuit, but maintains oscillation for values of the frequency of oscillation of the load circuit varying within a range having at least a 10% difference between minimum and maximum values of the range.

11. The non-thermal plasma generator of any of claims 1 to 10, wherein an operating voltage amplitude at the plasma generating site produced when the current is driven through the primary coil of the gain transformer is at least 1 kV RMS, optionally, wherein the at least one decoupling inductor together with the gain transformer comprise a one or more transformers delivering the operating voltage to the plasma generating site with an amplitude at least 20 times larger than a voltage amplitude in the primary coil of the gain transformer.

12. The non-thermal plasma generator of any of claims 1 to 11, comprising a transmission line interconnecting the plasma generating site and the at least one decoupling inductor; wherein the transmission line is at least 50 cm long, and flexible, optionally, provided together with a gas supply lumen leading along the transmission line to the plasma generating site, the gas supply lumen and transmission line together being elements of a flexible probe having an overall diameter of less than 10 mm.

13. The non-thermal plasma generator of any of claims 1 to 12, wherein the primary coil of the gain transformer has an inductance in the range of about 1-5 µH, and the secondary coil of the gain transformer has an inductance in the range of about 1000-5000 µH.

14. The non-thermal plasma generator of any of claims 1 to 13, wherein the at least one decoupling inductor includes at least one decoupling transformer, and a distal-side coil of the at least one decoupling transformer has an inductance in the range of about 20-80 µH, and is a coil of a distal decoupling transformer of the at least one decoupling transformer having a primary coil with an inductance in the range of about 5-20 µH.

15. The non-thermal plasma generator of any of claims 1 to 14, wherein the at least one decoupling inductor is connected to the plasma generating site through releasable electrical contacts.

FIG. 1A

FIG. 1B

FIG. 1C

FIG. 2A

FIG. 2B

300

202

301

305A

204

505

Dosimeter

203

| Input voltage | Primary coil driver circuit | Gain Transformer |  | Coaxial cable |
| --- | --- | --- | --- | --- |
|  |  | Decoupling Transformer |  |  |

305B

206

Plasma load

## FIG. 3A

320

Deliver input voltage to power driver circuit

322

Receive feedback signal response from load circuit comprising transmission line & its impedance

324

Entrain driver circuit to load circuit resonance via feedback signal

326

Boost driver circuit AC voltage to first-stage AC voltage via gain transformer

328

Generate second-stage AC voltage from first-stage AC voltage via decoupling transformer

330

Transmit second-stage AC voltage through transmission line to plasma generating site & generate plasma

## FIG. 3B

FIG. 3C

FIG. 3D

400

401

505

Dosimeter

202

204

305A

203

| Input voltage | Primary coil driver circuit | Gain Transformer | Coaxial cable |

Decoupling Transformers

405

206

Plasma load

FIG. 4A

FIG. 4B

EP 4 447 088 A1

300

202

301

305A

204

505

203

206

501

Input
voltage

Primary coil
driver
circuit

Gain
Transformer

Decoupling
Coil

Dosimeter

Coaxial
cable

Plasma
load

FIG. 5A

FIG. 5B

Plasma Load

206

203

$C_{coax}$

501

C6

C5

L4

L3

L1

L2

305A

300

301

204

308

306

C4

307

L_FB

C_FB

202

Europäisches
Patentamt

European
Patent Office

Office européen
des brevets

# EUROPEAN SEARCH REPORT

**Application Number**

EP 24 16 7544

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 773 288 A1 (CREO MEDICAL LTD [GB]) 17 February 2021 (2021-02-17) | 1,12-14 | INV. H01J37/32 H05H1/46 A61B18/04 |
| Y | * figures 2, 3 * | 2-7,11 | |
| A | * paragraph [0048] - paragraph [0071] * | 8-10,15 | |
| | ----- | | |
| Y | US 2020/254271 A1 (ECKERT BRADLEY N [US] ET AL) 13 August 2020 (2020-08-13) * figure 18 * * paragraph [0059] - paragraph [0078] * | 2-7,11 | |
| | ----- | | |
| A | WO 2004/014439 A2 (ACCESS BUSINESS GROUP INT LLC [US]; TAYLOR ROY M [US] ET AL.) 19 February 2004 (2004-02-19) * the whole document * | 1-15 | |
| | ----- | | |

|  | TECHNICAL FIELDS SEARCHED (IPC) |
|---|---|
|  | H01J H05H A61B |

The present search report has been drawn up for all claims

| Place of search | Date of completion of the search | Examiner |
|---|---|---|
| The Hague | 8 August 2024 | Hochstrasser, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 447 088 A1**

## ANNEX TO THE EUROPEAN SEARCH REPORT
## ON EUROPEAN PATENT APPLICATION NO.

EP 24 16 7544

08-08-2024

| Patent document cited in search report | | Publication date | Patent family member(s) | | Publication date |
|---|---|---|---|---|---|
| EP 3773288 | A1 | 17-02-2021 | AU | 2019245726 A1 | 23-07-2020 |
| | | | BR | 112020014008 A2 | 01-12-2020 |
| | | | CA | 3088331 A1 | 13-07-2020 |
| | | | CN | 111629680 A | 04-09-2020 |
| | | | EP | 3773288 A1 | 17-02-2021 |
| | | | ES | 2906407 T3 | 18-04-2022 |
| | | | GB | 2572400 A | 02-10-2019 |
| | | | IL | 275882 A | 31-08-2020 |
| | | | JP | 7402525 B2 | 21-12-2023 |
| | | | JP | 2021518770 A | 05-08-2021 |
| | | | KR | 20200136881 A | 08-12-2020 |
| | | | PT | 3773288 T | 17-02-2022 |
| | | | RU | 2020123242 A | 29-04-2022 |
| | | | SG | 11202006724P A | 28-08-2020 |
| | | | US | 2020397497 A1 | 24-12-2020 |
| | | | WO | 2019185331 A1 | 03-10-2019 |
| US 2020254271 | A1 | 13-08-2020 | US | 2020254271 A1 | 13-08-2020 |
| | | | US | 2023330427 A1 | 19-10-2023 |
| WO 2004014439 | A2 | 19-02-2004 | AU | 2003259043 A1 | 25-02-2004 |
| | | | CA | 2494940 A1 | 19-02-2004 |
| | | | CN | 1691966 A | 02-11-2005 |
| | | | EP | 1539256 A2 | 15-06-2005 |
| | | | JP | 2006503609 A | 02-02-2006 |
| | | | KR | 20050071466 A | 07-07-2005 |
| | | | TW | I264313 B | 21-10-2006 |
| | | | US | 2004140194 A1 | 22-07-2004 |
| | | | WO | 2004014439 A2 | 19-02-2004 |

EPO FORM P0459